# EUROPEAN PATENT APPLICATION

(11) **EP 3 791 884 A1**
(43) Date of publication of application: **17.03.2021**
(21) Application number: 19197581.2
(22) Date of filing: 16.09.2019
(51) Int. Cl.: A61K 31/727, A61K 33/14, A61P 31/12

(54) **COMBINATION OF HEPARIN AND MAGNESIUM SALT FOR THE TREATMENT OF VIRAL INFECTIONS**

(71) Applicant: Bunz, Oskar, 58452 Witten (DE); Mese, Kemal, 42283 Wuppertal (DE); Erhardt, Anja, 44141 Dortmund (DE)
(72) Inventor: Bunz, Oskar, 58452 Witten (DE); Mese, Kemal, 42283 Wuppertal (DE); Erhardt, Anja, 44141 Dortmund (DE)

(57) **Abstract**

A pharmaceutical or veterinary composition is disclosed, comprising heparin and one or more pharmaceutically acceptable magnesium salts. Further, the pharmaceutical or veterinary composition or a combination of heparin and one or more pharmaceutically acceptable magnesium salts for use in a prophylactic or therapeutic method for treating virus infections is disclosed. The treatment preferably is a topical treatment or intravenous treatment.

## Description

The present invention relates to a combination of heparin and a pharmaceutically acceptable magnesium salt, in particular magnesium chloride, and its use in a method for preventing and/or treating virus infections, in particular infections caused by human Adenovirus.

### Background of the invention

### Antiviral treatment in the clinic

Virus infections cause a wide range of symptoms. Most treatments which are available today are based on limiting the symptoms. Antiviral therapy is usually understood as therapy by applying drugs that inhibit the spread of the virus. Although research in the field of virology is gaining more and more basic knowledge about viruses and their properties in vivo, the number of antiviral drugs is limited with about 40 different kinds (about 30 against HIV). Most antiviral drugs were developed to combat human immunodeficiency virus (HIV) infections. Table 1 summarizes drugs and their antiviral mechanism used to combat virus infections in the clinic.

### Abbreviations used in Table 1:

HBV: Hepatitis B virus
HSV: Herpes simplex virus
VZV: Varicella zoster virus
CMV: Cytomegalovirus
EBV: Epstein Barr Virus

**Table 1: Antiviral treatment in the clinic (modified from T. Karow, Lang-Roth, 2017).**

| **Drug Class** | **Active Substance** | **Trade Name** | **Drug Approval** | **Mechanism** |
|---|---|---|---|---|
| **Non-Nucleoside Reverse Transcriptase Inhibitor** | Efavirenz | Sustiva® | HIV-1 | Binding at a remote location of the active RT |
| | Nevirapine | Viramune® | | |
| | Etravirine | Intelence® | | |
| | Rilpivirine | Edurant® | | |
| **Nucleoside analogue** *Thymidine analogue* | Zidovudine | Retrovir® | HIV-1 | Inhibition of reverse transcriptase (competitively) |
| | Stavudine | Zerit® | HIV-2 | |
| *Adenosine analogue* | Abacavir | Ziagen® | | |
| | Didanosine | Videx® | | |
| *Adenosinemonophosphate analogue* | Tenofovir | Viread® | HIV-1 | |
| | Adefovir | Hepsera® | HBV | Inhibition of the transcription from viral RNA into DNA |
| | Tenofovir | Viread® | | |
| *Cytidine analogue* | Emtricitabine | Emtriva® | HIV-1 | |
| | Lamivudin | Epivir® | HIV-2 | |
| | | Zeffix® | HBV | |
| *Guanosine analogue* | Acyclovir | Zovirax® | HSV | GTP-depletion: |
| | Valaciclovir | Valtrex® | VZV | |
| | Penciclovir | Fenistil® | | inhibits the binding of guanosin-nucleotides (GTP) |
| | Famciclovir | Famvir® | | |
| | Ganciclovir | Cymeven® | CMV | |
| | Valganciclovir | Valcyte® | | |
| | Entecavir | Baraclude® | HBV | |
| | Ribavirine | Copegus® | Respiratory Syncytial Virus HCV Arenavirus Adenovirus | RNA-mutation/error catastrophe: |
| *Thymidine analogue* | Brivudine | Zostex® | HSV-1 VZV | leads to a hypermutation of the viral RNA |
| | Telbivudine | Sebivo® | HBV | |
| *Cytidine monophosphate analogue* | Cidoforvir | Vistide® | CMV | |
| *Uridine monophosphate analogue* | Sofosbuvir | Sovaldi® | HCV (GT 1-4 | |
| **Fusion inhibitor** | Enfuvirtid | Fuzeon® | HIV | Avoids virus penetration |
| **M2 inhibitor** | Amantadine | PK-Merz® | Influenza A | Blocking of the M2 ion-channel |
| **Chemokine receptor antagonist** | Maraviros | Celsentri® | HIV | Fusion of HIV to host cell via CCR5 (penetration) |
| **Integrase inhibitor** | Raltegravir | Isentress® | HIV-1 | Prevents integration into host genome |
| | | | HIV-2 | |
| **Protease inhibitors** | Indinavir | Crixivan® | HIV-1 | Prevents HIV formation through |
| | | | HIV-2 | |
| | Nelfinavir | Viracept® | | |
| | Ritonavir | Norvir® | | competitive inhibition of the viral protease |
| | Saquinavir | Invirase® | | |
| | Atazanavir | Reyataz® | | |
| | Fosamprenavir | Telzir® | | |
| | Lopinavir | in Kaletra® | | |
| | Tipranavir | Aptivus® | | |
| **NS3/4A-Inhibitor** | Boceprevir | Victrelis® | HCV GT1 | Protein maturation inhibition |
| | Telaprevir | Incivo® | | |
| **DNA-Polymerase inhibitor** *Pyrophosphate analogue* | Foscarnet | Triapten® | HSV-1 | Inhibits DNA-Polymerase and RT |
| | | Foscavir® | CMV | |
| | | | VZV | |
| | | | EBV | |
| | | | HBV | |
| | | | HIV | |
| **Neuraminidase Inhibitor** | Oseltamivir | Tamiflu® | Influenza A+B | Inhibition of neuraminidase via binding to the active center of the enzyme |
| | Zanamvir | Relenza® | | |
| **Antibodies** *Specific immunoglobuline* | Cytomegalie-Ig | Cytotex® | | Passive immunization |
| | Hepatitis A-Ig | Beriglobin® | | |
| | Hepatitis B-Ig | Hepatect® | | |
| | Rabies-Ig | Berirab® | | |
| | VZ-Ig | Varitect® | | |
| *Therapeutic monoclonal antibodies (mAK)* | Palivizumab | Synagis ® | RSV | Humanized mAK |
| **Cytokines** | Interferon alfa-2a | RoferonA® | Chronic Hepatitis B/C | Interferon degrades viral mRNA and inhibits protein synthesis |
| | Interferon alfa-2b | IntronA® | | |
| | Peginterferon alfa-2b | Pegasys® | | |
| | Peginterferon alfa-2b | PegIntron® | Chronic Hepatitis C | |
| **T-cells** | | | | Ex vivo stimulating of virus specific T-cells. Also recombinant expression of T-cell receptors (TCR) |

One of the reasons for the limited possibilities to fight against virus infections is that, unlike bacteria or fungi, viruses amplify via the infected host cells. Agents against this mechanism often also act cytotoxic against the host cell (Ryu, 2016). Another important point is the fact that viruses have the capacity to mutate there genome more often than bacteria, which can lead to the development of drug resistances and vaccine failure (Ryu, 2016).

Viral DNA/RNA polymerases are well suited as targets for antiviral therapy. Most antiviral agents are DNA/RNA polymerase inhibitors. One of them, acyclovir, is the first generation of selective antiviral agents. Since its discovery in 1977, acyclovir has been used as a standard agent against Herpes simplex virus 1 (HSV-1), Herpes simplex virus 2 (HSV-2), and Varicella zoster virus (VZV) (Ryu, 2016).

Most of these drugs are nucleoside analogues. Unfortunately, the emergence of resistances limits the success of this therapy (Ryu, 2016).

In the following paragraph the different mechanisms of antiviral treatment options are mentioned and exemplified for common virus infections represented by herpesviruses, HIV, influenza virus and hepatitis C virus (HCV). Furthermore new mechanisms to combat virus infections are shortly mentioned including the monoclonal antibody treatment and the activation of the innate immune system through the antiviral toll-like receptor (Ryu, 2016). Looking at the possibilities of antiviral therapies it can be stated that various antiviral drugs are used for antiviral treatment in the clinic, but considering the diversity of human pathogenic viruses, there are also numerous viruses for which no treatment is available. One example for such viruses are adenoviruses, for which no specific antiviral therapies are available. In these cases, the therapy is based on case reports/off label uses.

### Heparin and heparan sulfate

Heparan sulfate proteoglycans (in the following abbreviated as HSPGs) play an important role in numerous mechanisms like cell migration, cell adhesion, cell growth and proliferation, tumorigenesis, angiogenesis, and cellular attachment of viruses. Important for these functions is the protein-binding capacity of HSPGs. The exact underlying mechanism of the interaction between HSPGs and proteins is not known, but it is speculated that the highly sulfated region plays an important role (Mulloy & Forster, 2000). However, it is established, that, for instance, different viruses like Herpes virus, Rift Valles Fever Virus, Sindbis Virus bind to HSPGs (Byrnes & Griffin, 1998; de Boer et al., 2012; Shukla & Spear, 2001).

Heparin, also referred to as unfractionated heparin (UFH), is mainly produced in human or mammal mast cells as heparin proteoglycan. Heparin possesses a core protein called serglycin and various heparin polysaccharide chains. The most common disaccharide unit is composed of a 2-O-sulfated iruronic acid and 6-O-sulfated, N-sulfated glucosamine, IdoA(2S)-GlcNS(6S). Up to 85 % of heparins in beef lung and about 75 % of heparins in porcine intestinal mucosa contain this particular disaccharide.

Heparin is a polymer with a molecular weight of 3,000 to 30,000 Dalton (Da), which is in contrast to most commercially available heparins with an average molecular weight ranging from 12,000 to 15,000 Da.

The structures of the disaccharide units contained in heparin sulfate and heparin are schematically shown in the following scheme.

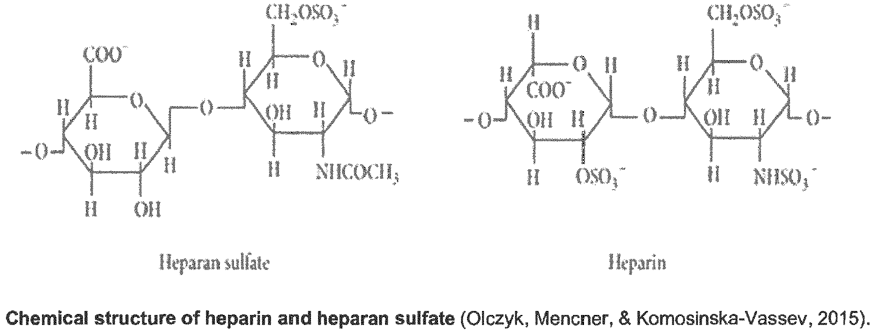

The three-dimensional structure of heparin is complicated because iduronic acid may be present in either of the two low-energy conformations when positioned within an oligosaccharide. The conformational equilibrium is influenced by the sulfation state of adjacent glucosamine sugars. Often, heparin adopts a helical conformation in which clusters of sulfate groups at regular intervals on either side of the helical axis can be present.

Heparin is well known and used in medicine. It is on the World Health Organization's list of Essential medicines. It is specifically used in heart attacks and unstable angina as well. It is given parenterally (injection into a vein or subcutaneously) because it is not absorbed from the intestine due to its high negative charge and large size. Because of its short biologic half-life of about one hour it must be given frequently or as a continuous infusion. Unfractionated heparin has a half-life of about one to two hours, whereas Low-Motecutar-Weight Heparins (LMWH, see below) has a half-life of four to five hours.

LMWH has an anticoagulant activity because of its binding to and activation of Antithrombin III (Mulloy & Forster, 2000). After binding to heparin, Antithrombin III shows changes in its conformation and forms complexes with Factor Xa and thrombin. Only the pentasaccharide chains with a certain amount of monosaccharides are able to build up a Antithrombin III and Factor Xa complex (Karpukhin, Feofanova, Nikolaeva, Mamontov, & Dobrynina, 2006).

Heparin shows defined glycosidic linkages in iduronate possessing sequences (Karpukhin et al., 2006). The glycosidic bond conformations are not sulfation-dependent and identical in all different forms of heparin. It is important to know that, even though the conformational equilibria of their iduronate residues are different, both heparin and heparin sulfate contain highly sulfated, iduronate-containing sequences. In heparin this is often the case and in heparan sulfate occasionally. Furthermore, both have a sequence (polysaccharide) with a high affinity to Antithrombin III. The interaction between heparin produced in mast cells and Antithrombin III is very special and not comparable to other heparin-protein interactions. This kind of heparin possesses a specific monosaccharide sequence, which has high affinity to Antithrombin III. Due to this sequence, heparin undergoes a conformational change after binding to Antithrombin III. Thus, after that binding and conformation change, Antithrombin III is more efficient as an inhibitor of its target serine proteases (Mulloy & Forster, 2000). Due its molecular structure heparin shows an unusual mobility because of the iduronate ring. Moreover, it shows an unusual rigidity because of its glycosidic conformations, which altogether makes heparin to be different from other known polysaccharides (Mulloy & Forster, 2000).

The functions of heparin are also very divers. Heparin can modulate tumor and metastasis growth. Furthermore, heparin activates the release of hepatic lipase and lipoprotein lipase, leading to an inhibition of the complement system and angiogenesis. Heparin controls several interactions between proteins and the cellular surface (Hu, Fink, Grant, & Elder, 2014).

### Low-Molecular-Weiqht Heparins (LMWH)

LMWH belong to the family of heparins. They also have an antithrombogenic effect and are used in the clinic for the therapy and prevention of embolic diseases. LMWH prolong the activated plasma thromboplastin time (aPTT) and inhibit activated factor X. Furthermore, clinical studies revealed a lower risk of bleeding as a positive effect of LMWH. The molecular size of LMWH ranges from 4,000 to 6,000 Da. There are different forms of LMWH, which differ in their molecular size, pharmacokinetic properties, composition of glycosaminoglycans and electrical charge. The mucopolysaccharides (GAGs) are composed of uronic acid and glycosamine and are difficult to distinguish from the body's own heparins. LMWH is mainly applied subcutaneously. As with unfractionated heparins, these are polysulphated uronic acid-amino sugar derivatives (polyanionic electrolytes). In vitro an anti-Xa and an antithrombin activity are observed. Bioavailability in subcutaneous application is higher than 90% and for unfractionated heparin (UFH) 10-29% at low dose. In contrast to UFH, where the pharmacokinetics are rather linear, the pharmacokinetics of LMWH are linear (Frydman, 1996). The heparin dose can be measured and monitored by aPTT measurements, whereas LMWH can only be monitored via the Anti-Xa-level. Protamine sulphate is used as an antidote against heparin and there is currently no antidote on the market against LMWH (Karow & Lang-Roth, 2012). Heparins having a chain length of 5 monosaccharides or more (three D-glucosamines and two uronic acids) have anticoagulant activity. These chain segments are negatively charged, by which they bind to Antithrombin III. Heparins having a chain length of 5 to 17 monosaccharides are designated LMWH, starting from a chain length of 18 they are designated unfractionated heparins (UFH). Commercially available heparins are e.g. Certoparin (5,400 Da), Dalteparin (6,100 Da), Enoxaparin (4,500 Da), Parnaparin (5,000 Da) Nadroparin (4,300 Da), Reviparin (4,400 Da) and Tinzaparin (6,500 Da) (Mutschler, 2012).

Enoxaparin-sodium is the active ingredient in the well-known anticoagulant Clexane® (Sanofi-Aventis). The molecular weight of the heparin in Clexane® is 4,500 Da. Clexane® is available in different strengths, such as 2,000 I.U (20 mg) per 0.2 ml solution, 4,000 I.U (40 mg) per 0.4 ml solution, 6,000 I.U. (60 mg) per 0.6 ml solution, 8,000 I.U. (80 mg) per 0.8 ml solution and 10,000 I.U. (100 mg) per 1.0 ml solution for injection, respectively.

The quantity of heparin is usually defined in I.U. (international units) which are commonly defined as follows: 1 international unit of heparin is the quantity of heparin required to prevent coagulation of 1 ml citrate-containing plasma after addition of CaCl₂ at 37 °C for 1 h.

### Heparin Protein Complexes

There are several proteins known to bind to heparin. The binding depends on the trisulfated disaccharide repeat unit and the iduronate rings. Therefore, heparin is able to bind to the same protein via these two mechanisms. It is speculated, that heparin and heparin sulfate act like catalysts of molecular interactions. Heparin induces binding between Antithrombin III and its serine protease targets. After binding the protein-protein complex is intact (Mulloy & Forster, 2000).

Heparin is able to bind to calcium, magnesium, zinc and copper (Kang, Lee, & Gorenstein, 2007; Stevic, Parmar, Paredes, Berry, & Chan, 2011) and there is evidence that it interacts with magnesium and other ions in the cell. In the case of heparin and interactions with bacteria, heparin/heparan sulfate plays an important role in the infection of the cell, which was demonstrated for Escherichia coli (E. coli) (Kang et al., 2007). It was shown that magnesium specifically inhibits the interaction between E. coli and heparin/heparan sulfate. In addition, it is known that the inhibition is not magnesium chloride- but magnesium-dependent. As magnesium is known as an ion with the highest density compared to all other ions in cells, magnesium binds to anionic sites, preferentially to polyphosphates. It is believed that the inhibition of the binding between E. coli and heparin is due to electroneutralization of the highly negatively charged heparin as a polymer caused by magnesium. The binding of magnesium to heparin is purely electrostatic (Kang et al., 2007).

It has been proposed that the neutralizing effect of calcium and magnesium on the anticoagulant action of heparin and antithrombin III may be related to the ability of these metals to prevent the complex formation of the acid mucopolysaccharide and the thrombin inhibitor by binding to the acid mucopolysaccharide. Magnesium was reported to have relatively high affinity for heparin and to bind to heparin at the same binding sites as calcium. It is assumed that binding of calcium and magnesium to heparin occurs at both the carboxylate and sulfate groups of heparin (Yamane et al., 1982).

### Heparin and Heparan sulfate and viruses

Some studies demonstrated an antiviral activity of heparin. In the case of HIV and feline immunodeficiency virus (FIV) heparin is able to inhibit the viral uptake in cells (Hu et al., 2014). Mechanistically this effect is due to the virus-HSPG interaction in vitro and it was demonstrated that heparin binds to the V3 loop of FIV. However, it was found that this function is reduced to laboratory adapted strains and the inhibition of virus entry does not work in the case of field isolates. Furthermore, there is evidence that heparin interferes with the surface glycoprotein (SU) which binds to the HIV co-receptor CXCR4 (Hu et al., 2014). Moreover, it is known that baculovirus uses heparin as a binding protein to enter mammalian cells (Wu & Wang, 2012). In case of human adenoviruses (HAdV) it was shown that the binding of the HAdV is not only caused by the loading of the heparin, but also by the heparan sulfate glycosaminoglycans (HS GAGs), which was demonstrated by performing binding assays (Dechecchi et al., 2001).

In case of the CSFV (Classical Swine Fever Virus), the interaction between heparin/heparan sulfate is due to the E(rns) protein, which is a mutated surface protein, appearing on the surface of these viruses after maintaining them in cell culture. Note that the virus directly obtained from the infected stock lacks binding to heparin (Hulst, van Gennip, & Moormann, 2000).

A second mechanism could be a protein-protein interaction between virus and heparin/heparan sulfate. Regarding HAdV the charge of the virus and heparin may play a main role in the interaction. It was previously published that human coagulation factor X (hFX) is necessary as a binding bridge for some adenoviruses to be able to enter into liver- and SKOV3 cells. Moreover, hFX has an affinity to heparan sulfate (Alba et al., 2009; Zaiss, Lawrence, Elashoff, Esko, & Herschman, 2011).

The HAdV types binding hFX are classified by Waddington and colleagues. In this publication HAdV 50 showed the highest binding affinity, followed by HAdV 2, 6, 5, 49 and 13, 11, 37, 35, 7, 3, 18, 46 (hFX binding affinity is listed from high to the lowest range). Binding affinities of HAdV to hFX are summarized in the following Table 2.

**Table 3: Affinities of human adenoviruses to the human coagulation factor X (hFX) (Waddington et al., 2008).**

| | Human Adenovirus Types |
|---|---|
| High affinity to hFX | 50, 16 |
| Moderate affinity to hFX | 49, 5, 6, 2 |
| Low affinity to hFX | 46, 18, 3, 7, 35, 37, 11, 13 |
| No affinity to hFX | 20, 29, 25, 17, 26, 28, 44, 48 |
| Affinity not known | 23, 1, 40, 31, 14, 34, 21, 4, 9, 32, 42, 51, 30, 15, 19, 10, 45, 27, 22, 8, 47, 24, 36, 38, 33, 39, 43 |

Note that DE 24 178 859 describes that heparin (in the unfractionated form and as LMWH) for medical purposes is usually available in the form of its sodium salts, which are generally obtained during isolation and purification from natural sources, in particular from the extraction of natural biological sources. Since heparin has a stronger affinity for calcium than for sodium, its medical application is often accompanied by bonding of calcium, which is extracted from different tissues of the organism and in particular locally from blood vessel and capillary walls. The document describes a method for obtaining heparin salts comprising metal cations other than sodium, or mixed heparin salts, their anticoagulant activity, and medicaments comprising such salts. The medicaments are in form of a solution which is applicable by intravenous, subcutaneous or intramuscularly. Heparin magnesium salts having an activity of 158 I.U./mg having a magnesium content of 5.4 % and 1.4 %, respectively, are disclosed. They are obtained from Codex-heparin as sodium salt of 160 I.U./mg. The method comprises two steps of metal exchange, e.g. by dialysis.

US 5,514,667 discloses compositions comprising a first component selected from loscarnet, suramin, sulfated polysaccharides such as heparin and polysulfated polysaccharides such as dextran polysulfate and others, and a second component selected from anti-inflammatory drugs such as non-steroidal and steroidal anti-inflammatories, for topical treatment of vital conditions or neoplastic disorders. It is mentioned that magnesium complexes and zinc complexes of polysulfated polysaccharides are preferred since both magnesium and zinc are known to assist in wound healing. Example 1 describes topical application of a cream comprising 0.5 g zinc pentosan polysulfate and 10 ml triethanolamine salicylate mixed with 90 ml sorbolene cream to a patient with extended superficial herpes erosions to the skin resistant to conventional therapy. Example 5 discloses topical treatment of herpes simplex with a cream comprising 1 wt.-% bufexamac and 0.5 wt.-% zinc pentosan polysulfate.

EP 0 012 115 discloses a preparation for topical application of virus infections comprising a pharmaceutically acceptable salt of an acidic sulfated polysaccharide or acidic sulfated polymer and zinc ions, and the use of the preparations for treating herpes virus infections, in particular herpes hominis (HVH) infections such as herpes genitalis or herpes dermatitis. Mixtures of ZnSO₄ x 7 H₂O with heparin sodium are disclosed. The heparin has an activity of 160 I.U./mg and is obtained from Biofac AS, Kopenhagen. The preparations may be creams, ointments, tinctures, aqueous solutions and in particular gels.

WO 88/07060 mentions an antiviral effect of heparin e.g. on herpes simplex virus.

### Human adenovirus

Adenoviruses (members of the family *Adenoviridae*) are medium-sized (90-100nm), nonenveloped (without an outer lipid bilayer) viruses with an icosahedral nucleocapsid containing a double stranded DNA genome. Their name derives from their initial isolation from human adenoids in 1953.

They have a broad range of vertebrate hosts; in humans, more than 80 distinct adenoviral serotypes were identified to cause a wide range of illnesses, from mild respiratory infections in young children (known as the common cold) to life-threatening multi-organ disease in people with a weakened immune system. Classification of *Adenoviridae* can be complex. In humans, there are human adenovirus types (HAdV-1 to 80) classified in seven species (Human adenovirus A to G).

Adenoviruses represent the largest known nonenveloped viruses. They are able to be transported through the endosome (i.e., envelope fusion is not necessary). The virion also has a unique "spike" or fiber associated with each penton base of the capsid that aids in attachment to the host cell via the receptor on the surface of the host cell.

The adenovirus genome is linear, non-segmented double-stranded (ds) DNA that is between 26 and 48 Kbp. Although this is significantly larger than other viruses in its Baltimore group, it is still a very simple virus and is heavily reliant on the host cell for survival and replication. An interesting feature of this viral genome is that it has a terminal 55 kDa protein associated with each of the 5' ends of the linear dsDNA. These are used as primers in viral replication and ensure that the ends of the virus' linear genome are adequately replicated.

Adenoviruses are unusually stable to chemical or physical agents and adverse pH conditions, allowing for prolonged survival outside of the body and water. These viruses are spread primarily via respiratory droplets, however they can also be spread by fecal routes. Research regarding the molecular mechanisms underlying adenoviral transmission provide empirical evidence in support of the hypothesis that cellular receptors for adenovirus and coxsackievirus (CARs) are needed to transport adenoviruses into certain naive/progenitor cell types.

Most infections with adenovirus result in infections of the upper respiratory tract. Adenovirus infections often appear as conjunctivitis, tonsillitis (which may look exactly like strep throat and cannot be distinguished from strep except by throat culture), an ear infection, or croup. Adenoviruses types 40 and 41 can also cause gastroenteritis. A combination of conjunctivitis and tonsillitis is particularly common with adenovirus infections. Some children (especially small ones) can develop adenovirus bronchiolitis or pneumonia, both of which can be severe. In babies, adenoviruses can also cause coughing fits that look almost exactly like whooping cough. Adenoviruses can also cause viral meningitis or encephalitis. Rarely, adenovirus can cause hemorrhagic cystitis (inflammation of the urinary bladder - a form of urinary tract infection - with blood in the urine). Most people recover from adenovirus infections by themselves, but people with immunodeficiency sometimes die of adenovirus infections, and - rarely - even previously healthy people can die of these infections. This may be because sometimes adenoviral infection can lead to cardiac disorders. For example, in one study, some cardiac samples of patients with dilated cardiomyopathy were positive for presence of adenovirus type 8.

Adenoviruses are often transmitted by expectorate, but can also be transmitted by contact with an infected person, or by virus particles left on objects such as towels and faucet handles. Some people with adenovirus gastroenteritis may shed the virus in their stools for months after getting over the symptoms. The virus can be passed through water in swimming pools that do not have enough chlorine in them. As with many other illnesses, good handwashing is one way to inhibit the spread of adenoviruses from one person to another. Only heat and bleach will kill adenoviruses on objects.

There are no proven antiviral drugs to treat adenoviral infections, so treatment is largely directed towards the symptoms (such as fever). The antiviral drug cidofovir has helped some of those patients who had severe cases of illness. However, the exact number of patients which were treated and to what degree the treatment was successful in particular with respect to complications or symptoms were not given in the source. A doctor may give antibiotic eyedrops for conjunctivitis, while awaiting results of bacterial cultures, and to help prevent secondary bacterial infections. Currently, there is no adenovirus vaccine available to the general public, but a vaccine is available for the United States military for types 4 and 7 to prevent severe respiratory tract infections such as persistent bronchiolitis, joint pain, dilatative cardiomyopathy or sudden hearing loss.

### Antiviral Treatment of Adenovirus

The in vitro antiviral efficiency of ganciclovir, cidofovir and ribavirin against several types of adenovirus was evaluated in previous studies (Matthes-Martin, Boztug, & Lion, 2013). Ribavirin shows an antiviral activity against HAdV species C, and limited activation against species A, B, D. In the clinic, however, the antiviral effect was not really significant (Matthes-Martin et al., 2013). Ganciclovir, a well-known prodrug which requires phosphorylation, is efficient against herpes virus but not against adenovirus (Matthes-Martin et al., 2013). To achieve a significant treatment success, the in vitro experiments show a half-maximal effective concentration (EC₅₀), three times higher than in plasma. Because of that ganciclovir is not a common drug used in antiviral therapy.

Cidofovir is, for the mentioned reasons, the primary antiviral drug in the treatment of infections caused by adenovirus in immunodeficiency patients (Matthes-Martin et al., 2013). Cidofovir is nephrotoxic and leads to reversible tubulopathy (Matthes-Martin et al., 2013). Although the success rates in treatment using Cidofovir are low in the clinic (Matthes-Martin et al., 2013) it is used in the clinic in case of acute infections in combination with intravenous immunoglobulins and ribavirin and donor lymphocyte infusions.

Recently, a method for high-throughput direct cloning and engineering of adenoviral genomes from different sources utilizing advanced linear-linear homologous recombination (LLHR) and linear-circular homologous recombination (LCHR) has been developed (Zhang, W. et al., 2017). By this method, a library of 34 cloned adenoviral genomes originating from clinical samples has been obtained. The 34 types of this library represent all seven Ad species known to infect humans. These viruses have been tagged with reporter genes allowing the further characterization of these recombinant viruses. A 2A peptide multicistronic TurboGFP, NanoLuc® Luciferase (Promega) and kanamycin/neomycin selection marker (GLN) have been used for tagging.

Some available antiviral medicaments, in particular anti-HIV-medicaments, are extremely expensive, such as several hundreds of Euros per month for products such as Isentress®, Trizivir® or Viread®.

For adenovirus infections, in particular, there is no prophylactic or therapeutic medicament available.

In view of this situation, the object of the invention is to provide an effective prophylaxis and/or treatment options for virus infections including adenoviruses, which are readily available and therefore economically favorable.

### Non-patent Literature

Alba, R., Bradshaw, A. C., Parker, A. L., Bhella, D., Waddington, S. N., Nicklin, S. A., ... Baker, A. H. (2009). Identification of coagulation factor (F)X binding sites on the adenovirus serotype 5 hexon: effect of mutagenesis on FX interactions and gene transfer. Blood, 114(5), 965-971. doi:10.1182/blood-2009-03-208835
Byrnes, A. P., & Griffin, D. E. (1998). Binding of Sindbis Virus to Cell Surface Heparan Sulfate. Journal of Virology, 72(9), 7349-7356.
de Boer, S. M., Kortekaas, J., de Haan, C. A. M., Rottier, P. J. M., Moormann, R. J. M., & Bosch, B. J. (2012). Heparan Sulfate Facilitates Rift Valley Fever Virus Entry into the Cell. Journal of Virology, 86(24), 13767-13771. doi:10.1128/jvi.01364-12
Dechecchi, M. C., Melotti, P., Bonizzato, A., Santacatterina, M., Chilosi, M., & Cabrini, G. (2001). Heparan sulfate glycosaminoglycans are receptors sufficient to mediate the initial binding of adenovirus types 2 and 5. J Virol, 75(18), 8772-8780.
Esko, J. D., Stewart, T. E., & Taylor, W. H. (1985). Animal cell mutants defective in glycosaminoglycan biosynthesis. Proc Natl Acad Sci U S A, 82(10), 3197-3201.
Frydman, A. (1996). Low-molecular-weight heparins: an overview of their pharmacodynamics, pharmacokinetics and metabolism in humans. Haemostasis, 26 Suppl 2, 24-38. doi:10.1159/000217270
Hu, Q. Y., Fink, E., Grant, C. K., & Elder, J. H. (2014). Selective interaction of heparin with the variable region 3 within surface glycoprotein of laboratory-adapted feline immunodeficiency virus. PLoS One, 9(12), e115252. doi:10.1371/journal.pone.0115252
Hulst, M. M., van Gennip, H. G. P., & Moormann, R. J. M. (2000). Passage of Classical Swine Fever Virus in Cultured Swine Kidney Cells Selects Virus Variants That Bind to Heparan Sulfate due to a Single Amino Acid Change in Envelope Protein Erns. Journal of Virology, 74(20), 9553-9561. doi:10.1128/jvi.74.20.9553-9561.2000
Kang, J., Lee, M. S., & Gorenstein, D. G. (2007). Magnesium ion is an effective inhibitor of the binding of Escherichia coli to heparin. J Microbiol Methods, 71(3), 340-342. doi:10.1016/j.mimet.2007.10.002
Karow, T., & Lang-Roth, R. (2012). Allgemeine und spezielle Pharmakologie und Toxikologie, 21. Aufl. Selbstverlag, Pulheim, S, 681*.*
Karpukhin, L. E., Feofanova, M. A., Nikolaeva, L. S., Mamontov, M. N., & Dobrynina, N. A. (2006). Complexation of magnesium and calcium ions with heparin. Russian Journal of Inorganic Chemistry, 51(6), 908-914. doi:10.1134/s0036023606060106
Matthes-Martin, S., Boztug, H., & Lion, T. (2013). Diagnosis and treatment of adenovirus infection in immunocompromised patients. Expert Rev Anti Infect Ther, 11(10), 1017-1028. doi:10.1586/14787210.2013.836964
Mulloy, B., & Forster, M. J. (2000). Conformation and dynamics of heparin and heparan sulfate. Glycobiology, 10(11), 1147-1156.
Olczyk, P., Mencner, L., & Komosinska-Vassev, K. (2015). Diverse Roles of Heparan Sulfate and Heparin in Wound Repair. Biomed Res Int, 2015, 549417. doi:10.1155/2015/549417
Ryu, W.-S. (2016). Molecular Virology of Human Pathogenic Viruses: Academic Press.
Shukla, D., & Spear, P. G. (2001). Herpesviruses and heparan sulfate: an intimate relationship in aid of viral entry. J Clin Invest, 108(4), 503-510. doi:10.1172/jci13799
Stevic, I., Parmar, N., Paredes, N., Berry, L. R., & Chan, A. K. C. (2011). Binding of Heparin to Metals. Cell Biochemistry and Biophysics, 59(3), 171-178. doi:10.1007/s12013-010-9129-5
Waddington, S. N., McVey, J. H., Bhella, D., Parker, A. L., Barker, K., Atoda, H., ... Baker, A. H. (2008). Adenovirus serotype 5 hexon mediates liver gene transfer. Cell, 132(3), 397-409. doi:10.1016/j.cell.2008.01.016
Wu, C., & Wang, S. (2012). A pH-sensitive heparin-binding sequence from Baculovirus gp64 protein is important for binding to mammalian cells but not to Sf9 insect cells. J Virol, 86(1), 484-491. doi:10.1128/JVI.06357-11
Yamane, Y., Saito, S., & Koizumi, T. (1983). Effects of calcium and magnesium on the anticoagulant action of heparin. Chem Pharm Bull (Tokyo), 31(9), 3214-3221.
Zaiss, A. K., Lawrence, R., Elashoff, D., Esko, J. D., & Herschman, H. R. (2011). Differential effects of murine and human factor X on adenovirus transduction via cell-surface heparan sulfate. J Biol Chem, 286(28), 24535-24543. doi:10.1074/jbc.M111.241562

### Detailed description of the invention

The object of the invention is solved by the pharmaceutical or veterinary composition according to claims 1 to 6 comprising heparin and one or more pharmaceutically acceptable magnesium salts.

The invention further provides the pharmaceutical or veterinary composition or a combination of heparin and one or more pharmaceutically acceptable magnesium salts for us in a prophylactic or therapeutic method for treating virus infections.

The combination of heparin with a magnesium salt and the use thereof as an antiviral treatment according to the invention is new.

Heparin is already known as an antiviral substance and has been tested and described for its antiviral effect in various publications. However, since the antiviral effect is not strong, it is merely considered a "side effect" of lowered blood coagulation.

The antiviral effect of magnesium chloride has also been published in several journals. But as the inventors have shown in the Examples, the antiviral effect of magnesium chloride alone is not always given.

According to the invention, however, a maximum antiviral effect can be achieved by mixing heparin and one or more magnesium salts, which was shown to be effective against a variety of different viruses.

The interactions of other divalent cations with heparin cannot be used in clinical application for systemic therapy. Neither iron nor copper can be used in clinical applications without fear of complications such as dangerous reactions or deposits. In case of calcium, systemic application without monitoring is also not possible, as intravenous administration of calcium can lead to dangerous cardiac arrhythmias.

To the contrary, magnesium has the advantage that its systemic application is harmless. Furthermore, magnesium and calcium bind the iduronic acid chain and thus interfere with the anticoagulative effect of heparin. Therefore, by the composition of the invention physiological coagulation could be less affected compared to heparin without magnesium or calcium activation (Yamane, Saito, & Koizumi, 1983).

The inventors have also used zinc for some test series (not described in the Examples section). Here, zinc showed a very strong cytotoxic effect. Furthermore, the antiviral effect of zinc was not apparent for all adenoviruses tested. An antiviral effect of zinc was detectable only for adenovirus type 5. Adenovirus type 10 even resulted in increased infection efficiencies in cells treated with zinc.

Consequently, as shown by the inventors, zinc in combination with heparin is not suitable for antiviral therapy or prophylaxis.

All areas of virus control are suitable as areas of application and timing for the combination according to the invention. Even after an infection, viral proteins continue to multiply, which ultimately leads to acute symptoms. The production of viral particles can lead to cell death and thus to the release of viruses. Here the inventors see an approach for therapy with the inventive combination of heparin and magnesium salt, because this cycle is expected to be broken by preventing the infection of further cells and contributing to a reduction of the viral load. The body of the patient gains at least time associated with lower infectivity of the viral particles and thereby the chance to recover from the infection and constant burden of virus production.

Preferably, the composition of the invention is in form of a solution. Water is the preferable solvent since heparin and magnesium salts are easily soluble in water, but other polar solvents may equally be used provided they are not toxic in topical treatments. Such solvents are, for example, alcohols such as methanol, ethanol, n-propanol, isopropanol, t-butanol, sec-butanol, n-butanol, acetone, ethyl acetate, methylethylketone, DMSO, DMF, or any other pharmaceutically acceptable polar solvent.

Since heparin is not resorbed enterally, the composition is used as a topical formulation or applied intravenously (i. v.) as solution for systemic application. For example, the composition can be formulated as solution for injection, gel, cream, ointment, spray, aerosol, tincture, eyedrops, nose drops, nose spray, solution or lotion. Flushing of hollow organs, rectal and vaginal flushings, and treatments of infections in the urinary bladder, uterus, ear, eye, and the oral cavity are feasible by topical application.

One or more pharmaceutically acceptable excipients and/or auxiliary agents are usually comprised in the composition, which are well-known to the skilled person in the field of pharmacy, such as base creams, flavors, preservatives, sweeteners, emulsifiers, pH adjusting agents, thickeners, surfactants, antioxidant, UV stabilizers, and the like.

Heparin can be used in any molecular weight distribution, but usually pharmaceutical preparations of heparin are preferable, such as UFH or LMWH as explained above. The molecular weight of UFH according to the invention is 4,000 to 40,0000, preferably 12,000 to 15,000 Da, and that of LMWH is 4,000 to 6,000 Da. One or more types of heparin can be used in combination. Heparin can be used advantageously as heparin sodium in the form provided by the pharmaceutical manufacturer.

The ratio of heparin to magnesium salt in the composition of the invention is preferably in the range of 25 I.U heparin : 1 µmol magnesium salt to 125 I.U. heparin : 10 µmol magnesium salt. Most preferably, the composition of the invention contains heparin and magnesium salt in a ratio of 5 I.U. heparin : 5 µmol Mg.

Commercially available heparin preparations having an activity specified by the manufacturer are used as stock solution and diluted to the desired activity.

The composition comprising heparin and one or more magnesium salts can be used in a therapeutic or prophylactic method for treating virus infections, or heparin and magnesium salt can be combinedly used in such method. In the latter case, the components can be used concomitantly or successively in any order. For example, the magnesium salt is applied first, e.g. as a solution, and then within a time interval of 30 s to 1 hour, heparin is applied as a solution, or vice versa.

The treatment can be applied before the patient or animal comes into contact with the virus, or after an assumed contact with virus without manifest infection, or after a virus infection has become manifest.

The treatment can be applied once per day or up to several times per day, according to need.

The virus infection which may be treated according to the invention are preferably infections caused by adenovirus, such as respiratory tract disorders (e.g. cold, bronchitis, pneumonia, acute respiratory distress syndrome), gastroenteritis, keratoconjunctivitis epidemica, cystitis, rhinitis, pharyngitis or diarrhea.

The inventive composition has been shown to be effective against various types of adenovirus, namely HAdV 3, 16, 21, 50, 5, 9, 10, 20, 24, 33, 37, 69 and 4.

Further, it is effective against HSV-1 and H9N2, therefore, the inventive composition can be used to treat herpes simplex infections and infections caused by influenza virus.

It is expected that, due to the mechanism of action which is based on electric charges, viruses do not form resistances against the inventive composition, other than in the case of conventionally known antivirus medications.

### Description of the Figures

Figure 1 shows the effects of different concentrations of heparin, Clexane® and MgCl₂ on human adenovirus type 5 (HAdV5) infectivity in CHO-K1 cells.
Figure 2: Adenovirus infection of adenovirus receptor encoding CHO cells and A549 cells in the presence of heparin and MgCl₂.

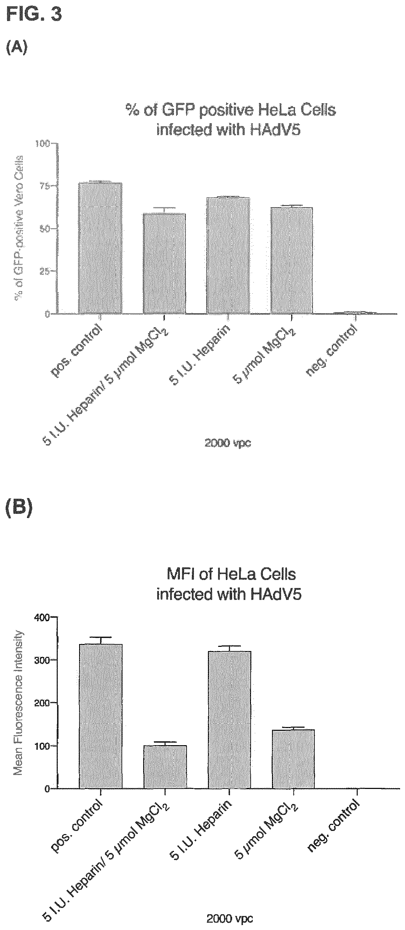

Figure 3 depicts inhibiting effects of heparin (5 I.U./ml) and MgCl₂ (5 µmol/ml) in HeLa cells investigated by flow cytometry analyses.
Figure 4 shows the effect of HSPG-deficiency on adenovirus infectivity in the presence and absence of heparin and MgCl₂ exemplified by different adenovirus.
Figure 5 shows results of ELISA experiments to measure the binding of IVIG to heparin- and MgCl₂-coated adenovirus virions.
Figure 6 depicts effects of different concentrations of heparin on HAdV infectivity in HSPG-deficient cells.
Figure 7: A direct comparison of HAdV infectivity in CHO-pgsE-606 and CHO-psgA-745 cells.
Figure 8: Coagulation factor X (FX) competition assays in SKOV3 cells using heparin / MgCl₂ covered HAdV virions.
Figure 9: In-depth analyses of different HAdV5 (V) infection and incubation conditions with heparin (H) and MgCl₂.
Figure 10: Effects of incubation times and treatment with heparin (H) and MgCl₂ on HAdV10 (V) infectivity in CHO-K1 cells.
Figure 11: Measurements of HSV-1 on Vero cells showed decreased transduction efficiencies after treatment with Heparin/MgCl₂.
Figure 12: Immunofluorescence analyses of H9N2 infected MDCKII cells.

### Examples

Cell lines used in the following Examples were obtained from Andre Lieber, Washington University, Seattle, USA or from ATCC®. Cells were cultured in media as mentioned in the following Table:

| **Cell line** | | **Medium** | **Reference/Source** |
|---|---|---|---|
| CHO-K1 | Chinese hamster ovary cells | DMEM + 10% FBS 1% Pen/Strep | Andre Lieber, Washington University, Seattle, USA |
| CHO-pgsA-745 | Chinese hamster ovary cell mutant deficient in xylosyltransferase | DMEM + 10% FBS 1% Pen/Strep | Andre Lieber, Washington University, Seattle, USA |
| CHO-pgsE-606 | Chinese hamster ovary cell mutant partially deficient in heparin sulfate N sulfotransferase | DMEM + 10% FBS 1% Pen/Strep | Andre Lieber, Washington University, Seattle, USA |
| CHO-CAR | Chinese hamster ovary cells stably expressing coxsackie and adenovirus receptor (CAR | DMEM + 10% FBS 1% Pen/Strep, G418 1 mg/ml | Andre Lieber, Washington University, Seattle, USA |
| CHO-CD46-C2 | Chinese hamster ovary cells stably expressing CD46 receptor, C2 isoform | DMEM + 10% FBS 1% Pen/Strep, G418 1mg/ml | Andre Lieber, Washington University, Seattle, USA |
| A549 | Human lung epithelial cell line | DMEM + 10% FBS 1% Pen/Strep | ATCC® CCL-185™ |
| HeLa | Human epithelial cervix cancer cell line | DMEM + 10% FBS 1% Pen/Strep | ATCC® CCL-2™ |
| SKOV-3 | Human ovary cancer cell line | RPMI, 5% FBS, 1% Pen/Strep/Glutamine | ATCC® HTB-77™ |
| Vero | African green monkey kidney cells | DMEM + 10% FBS 1% Pen/Strep | ATCC® CCL-81™ |
| MDCK.2 | Canine epithelial-like kidney cells | DMEM + 10% FBS | ATCC® CRL-2936™ |

Heparin sodium was obtained from Ratiopharm. A solution having an activity of 5,000 I.U./ml was used as starting reagent.

For example, 1 µl of this heparin solution (corresponding to 5 I.U. heparin) was used and mixed with 10 µl MgCl₂ stock solution (0.5 M), i.e. 5 µmol MgCl₂. That is, absolute amounts of 5 I.U. heparin and 5 µmol magnesium chloride were added to each well, respectively. The obtained mixture is sometimes designated reagent solution in the following.

That is, the final concentrations of heparin and magnesium salt in the reagent solution were 0.45 I.U./ml and 4.5 µmol/ml, respectively.

Each well originally contained 100 µl cell-containing medium before addition of the reagent solution. The final concentration of heparin and magnesium salt per well is then 0.045 I.U./ml and 0.45 µmοl/ml, respectively.

In experiments using other amounts of heparin and magnesium salt, the amounts of heparin solution and magnesium chloride solution were adapted correspondingly.

Clexane® was obtained from Sanofi-Aventis. 1 ml contains 100 mg Enoxaparin sodium corresponding to 10,000 I.U.

The adenovirus types containing the luciferase and GFP reporter genes used were obtained from the adenovirus virus library developed by Zhang et al. (2017) from the group of one of the inventors, A. Ehrhardt, at Institute of Virology and Microbiology, Center for Biomedical Education and Research (ZBAF), Department of Human medicine, Faculty of Health, Witten/Herdecke University, D-58453 Witten, Germany.

The herpes virus HSV-1 (provided by Fraunhofer Institute for Interfacial Engineering and Biotechnology, D-70569 Stuttgart, Germany) used was only marked with a GFP reporter gene (green fluorescent protein) and was examined by using fluorescence-activated cell sorting (FACS).

The influenza virus H9N2 used was obtained from the Faculty of Veterinary Medicine, Institute of Virology, Center for Infectious Diseases, University of Leipzig, D-04103Leipzig, Germany.

The luciferase assay Nano-Glo® Luciferase Assay System is commercially available from Promega. Adherent cells are lysed and furamizine is oxidized by luciferase to yield furimamide while emitting light. The bioluminescence developed due to this reaction can be detected. Since the luciferase reporter gene is located in the virus, luminescence detection can be used as indicator for transduction efficiency. The assay Nano-Glo® Luciferase Assay has been carried out according to the instruction manual provided by the manufacturer.

A microplate reader GENios from Tecan, Zurich, Switzerland was used.

Adenovirus particle concentrations were determined by measuring the optical density at 260 nm and expressed as viral particles (vps) per milliliter (Jager et al., 2009), vpc means "viral particles per cells" and is defined as the viral particle number (vps) applied per cell.

Further materials used in the Examples are specified in the following Table:

| **Chemicals** | **Producer/Company** | **Composition** |
|---|---|---|
| Ampuwa® | Fresenius, Bad Homburg, Germany | 100% |
| DMEM cell medium | PAN Biotech, Aidenbach, Germany | 4.5 g/L Glucose, L-Glutamine, Sodium Pyruvate, 3.7g/L NaHCO₃ |
| Dimethylsulfoxid (DMSO) | Roth, Dautphetal-Buchenau, Germany | ≥99.5% |
| DPBS (Dulbecco's phosphate buffered saline) | PAN Biotech, Aidenbach, Germany | without Ca and Mg |
| Fetal Bovine Serum (FBS) | PAN Biotech, Aidenbach, Germany | Standard |
| Penicillin/Streptomycin | PAN Biotech, Aidenbach, Germany | 10,000 Units Penicillin/ml, 10 mg Strep/ml |
| G418 (Geneticin) | ThermoFisher, Waltham Massachusetts USA | 50 mg/ml |
| Trypsin-EDTA | PAN Biotech, Aidenbach, Germany | 0.05/0.02% in PBS |
| TPCK-Trypsin, Trypsin from bovine pancreas | Sigma, St. Louis, USA | |
| MgCl₂ | Roth, Dautphetal-Buchenau, Germany | 0.5 M |
| Heparin-Natrium | ratiopharm, Ulm, Germany | 5,000 I.U./ml |
| Clexane® | Sanofi-aventis, Paris, Fance | 100mg/ml |
| Coating buffer for ELISA | Produced in the laboratory | 0.2 M Na₂CO₃/ NaHCO₃ pH: 9.5 |
| Blocking buffer for ELISA | Produced in the laboratory | 3% BSA in PBS |
| Washing buffer for ELISA | Produced in the laboratory | 0.05% Tween 20 in PBS |
| Substrate solution for ELISA | Produced in the laboratory | Na₂HPO₄/Na-Citrate/dd H₂O (pH 5) |
| OPD Peroxidase Substrate | Sigma, St. Louis, USA | Tablet for the substrate solution |
| Stop solution for ELISA | Sigma, St. Louis, USA | 1M H₂SO₄ |
| Nano-Glo® Luciferase Assay System | Promega, Mannheim, Germany | |
| Human factor X | HTI (Haematologic Technologies), Essex, USA | Sizes: 100 µg, 1 mg 50% glycerol/water (v/v) |

| **Antibody** | **Producer/Company** | **Reference/Source** |
|---|---|---|
| Goat Anti-Human IgG H&L | Abcam, Cambridge, United Kinqdom | Second Antibody (1:2000 diluted) |
| Rabbit anti-nucleoprotein (NP) polyclonal primary antibody PA5-32242 | Thermo scientific, Germany | 1:750 |
| Alexa Fluor® 488 Goat Anti-Rabbit IqG | Life technologies, Germany | |
| Privigen IVIG | Privigen, Hattersheim am Main, Germany | Concentrated human serum-mix |

### Example 1: The Effects of Heparin, Clexane® and MgCl₂ on the Infectivity of Human Adenoviruses

Chinese Hamster Ovary cells (CHO-K1 cells) were cultured using 5 % CO₂ and 37 °C in tissue culture dishes until confluency was reached. The medium for treatment was DMEM medium (PAN-Biotech GmbH, Aidenbach, Germany) with 10 % FCS (PAN-Biotech GmbH, Aidenbach, Germany) and 1 % Penicillin/Streptomycin (PAN-Biotech GmbH, Aidenbach, Germany). Cells were harvested with trypsin after washing them with DPBS. Cells were counted by hemocytometer and microscope and seeded at a density of 3x10⁴ per well in 96-well tissue culture plates in triplicate for each test. All luciferase measurements were performed 26 hrs after infection. 50 µl medium of the cells were mixed with 50 µl solution from Nano-Glo® Luciferase Assay System. For each experiment a positive control (infected CHO cells without heparin and MgCl₂) and a negative control (non-infected cells) were included.

To test the effect on infectivity of heparin only, cells were seeded with heparin added directly to the culture medium. After the cells attached HAdV5 was added at (1000 vpc).

For the different test series cells were incubated in medium with the addition of heparin alone (2.5, 5, 7.5, 10, 12.5 I.U. per well). For direct comparison, a test series with Clexane®, the low molecular weight heparin (M_{w} 4,500 g/mol), was performed using the identical amount of international units. The procedure was the same.

To test the antiviral effect of MgCl₂ alone, cells were incubated in medium containing MgCl₂ in different amounts (1.25, 2.5, 3.75, 5, 6.25 µmol per well). After cell attachment, virus infection was conducted.

To test the effect of heparin and MgCl₂ on viral infection efficiencies a stable concentration of MgCl₂ per well (5 µmol per well) was used. MgCl₂ was added to the medium before culturing the cells, respectively. Heparin was used at different amounts (2.5, 5, 7.5, 10, 12.5 I.U. per well, respectively).

To test whether there is a general effect of heparin and MgCl₂ on HAdV infectivity, increasing amounts of heparin in combination with stable amounts of MgCl₂ were evaluated. Components (HAdV, heparin, MgCl₂) were mixed in tissue culture medium for CHO-K1 cells and added to the cells.

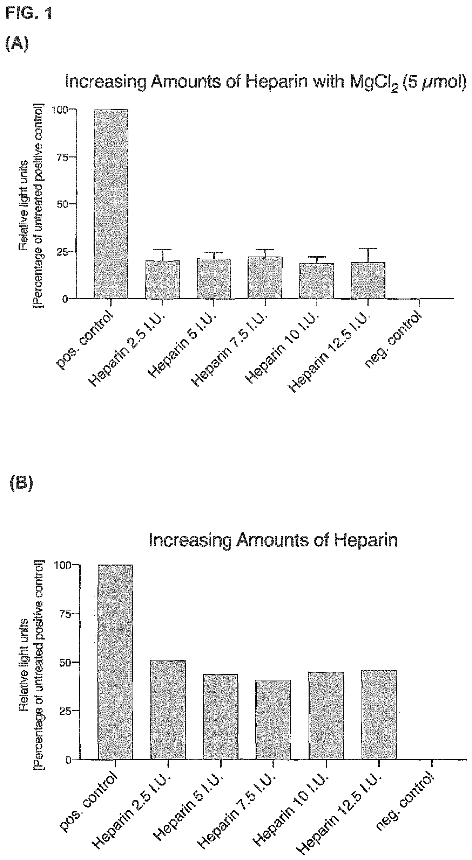

26 hrs post-infection luciferase assays were performed to evaluate the effect on HAdV infectivity, which directly correlate with luciferase values. With the addition of heparin alone and an applied viral dose of 1000 vpc a decrease from 100 % infectivity (i.e. luciferase value) to about 40 % was observed (Figure 1B). The strongest effect was measured with 5 and 7.5 I.U. heparin additions to each well containing 100 µl tissue culture medium.

Importantly, with the addition of MgCl₂ at a concentration of 5 µmol, a significant increase in the antiviral effect of heparin was measured. The luciferase values dropped to up to 20 % with the greatest effect after adding 2.5 I.U. and 10 I.U. heparin per well (Figure 1A).

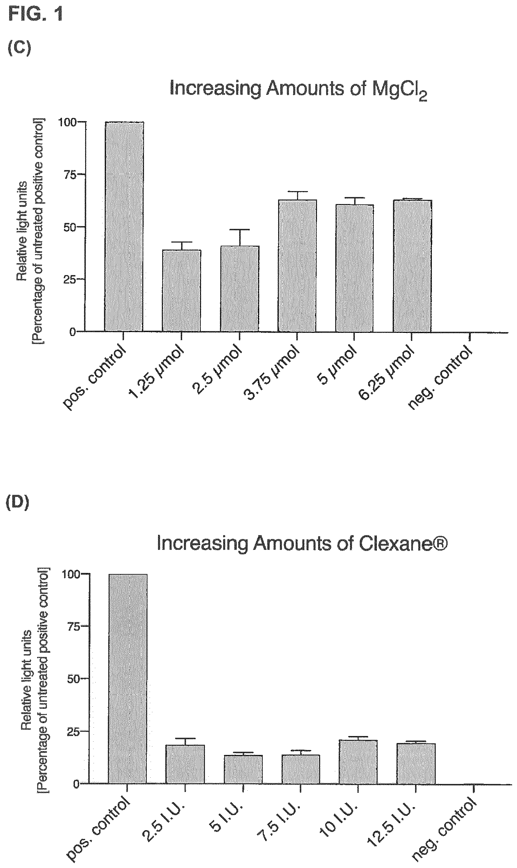

In the control group, which solely received MgCl₂ in different amounts and 1000 vpc of HAdV5, a moderate decrease of the luciferase values down to 40 % (with 25 µmol MgCl₂) was observed (Figure 1C).

For direct comparison, a test series with Clexane®, the low molecular weight heparin, was performed using the identical amounts of international units. Results are summarized in Figure 1D which revealed that the highest antiviral effect can be measured at 5 I.U., which was slightly lower than 20 % as compared to the positive control. This effect is comparable the observed effects when heparin in combination of MgCl₂ is applied at a dose of 7.5 I.U. (Figure 1B).

For the following experiments, a heparin dose of 5 I.U. was applied to analyze the anti-adenoviral effect.

### Example 2: Antiviral Effect of Heparin and MgCl₂ on Different Human Cell Lines and CHO Cell Lines Expressing Known Adenovirus Receptors

For these experiments A549, CHO-CAR and CHO-CD46 cells were used. The cells were cultured using 5 % CO₂ at 37°C in tissue culture dishes until confluency was reached. The medium for treatment was DMEM medium (PAN-Biotech GmbH, Aidenbach, Germany) with 10 % FCS (PAN-Biotech GmbH, Aidenbach, Germany) and 1 % Penicillin/Streptomycin (PAN-Biotech GmbH, Aidenbach, Germany). For selection, we added 100 µl Geneticin (50 mg/ml) to the culture medium of both CHO cells lines grown in 10 cm dishes with 10 ml medium. Cells were harvested with trypsin after washing with DPBS. Cells were counted and seeded at a density of 3x10⁴ per well in 96-well tissue culture plates in triplicates for each test. All luciferase measurements were performed 26 hrs post-infection with HAdV5 and HAdV50. Heparin and MgCl₂ were added before the infection to the culture medium.

CHO-CAR and CHO-CD46 cells stably encoding the CAR or CD46 receptor and A549 cells were infected with HAdV5 and/or HAdV50 (1000 vpc) in the presence of heparin (5 I.U. per well) and MgCl₂ (5 µmol per well). 26 hrs post-infection a luciferase assay was performed. 50 µl medium of the cells were mixed with 50 µl solution from the Nano-Glo® Luciferase Assay system. After five minutes incubation the cell lysate was used for performing the luciferase measurement. For each experiment untreated (no heparin and/or MgCl₂) control cells were analyzed.

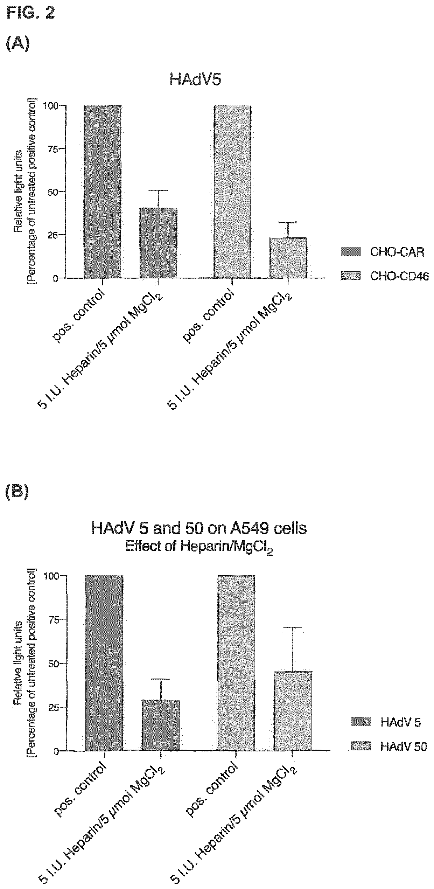

CHO-CAR cells stably encoding the CAR (Coxsackie Adenovirus Receptor) receptor were infected with HAdV5 (1000 vpc). Heparin (5 I.U. per well) and MgCl₂ (5 µmol per well) were added to the cell culture medium pre-infection and 26 hrs post-infection a luciferase assay was performed. This assay revealed a decrease of HAdV-derived luciferase expression levels from 100 % to 40 % (Figure 2A).

The identical experiment was performed with CHO-CD46 cells stably expressing the adenovirus receptor human CD46. Here a decrease from 100 % to approximately 20 % in luciferase expression levels was measured in the presence of heparin and MgCl₂.

These experiments show that there is an antiviral effect on CHO cells even in the presence of highly expressed known adenovirus receptors on the cell surface.

Next, human A549 cells derived from lung were analyzed because these cells are susceptible to HAdV infection and a majority of adenoviruses can replicate in these cells. For this experiment cells were treated first with heparin (5 I.U. per well) and MgCl₂ (5 µmol per well) and subsequently infected with HAdV5 (1000 vpc). Reduced luciferase levels were observed which decreased from 100 % to approximately 30% (Figure 2B). The same experimental setup was repeated with HAdV50 and a decrease of the luciferase protein measurement from 100% down to approximately 40 % was measured (Figure 2B).

### Example 3: HeLa Cells Infected with HAdV5 in the presence of inhibitory agents

In the following experiments HeLa cells were explored which are susceptible to adenovirus infection. HeLa cells were cultured using 5 % CO₂ and 37 °C in tissue culture dishes until confluency was reached. The medium for culturing was DMEM medium (PAN-Biotech GmbH, Aidenbach, Germany) with 10% FCS (PAN-Biotech GmbH, Aidenbach, Germany) and 1% Penicillin/Streptomycin (PAN-Biotech GmbH, Aidenbach, Germany). Cells were harvested with trypsin after washing them with DPBS, counted and seeded at a density of 1.5x10⁵ per well in 96-well tissue culture plates in triplicates for each test. All flow cytometry analyses were performed 26 hrs after infection. HeLa cells were infected with HAdV5 (1000 vpc).

Prior to infection (4 hrs) when plating the cells, cells were incubated with heparin (5 I.U. per well) and MgCl₂ (5 µmol per well) and heparin together with MgCl₂ at the same concentration (heparin 5 I.U. per well, MgCl₂ 5 µmol per well). Note that the reporter-tagged HAdVs also encode GFP besides luciferase and, therefore for these experiments flow cytometry analyses was performed 26 hrs post infection. For the measurement, cells were harvested after treating them with trypsin-EDTA. Cells were washed three times with DPBS before performing the measurement.

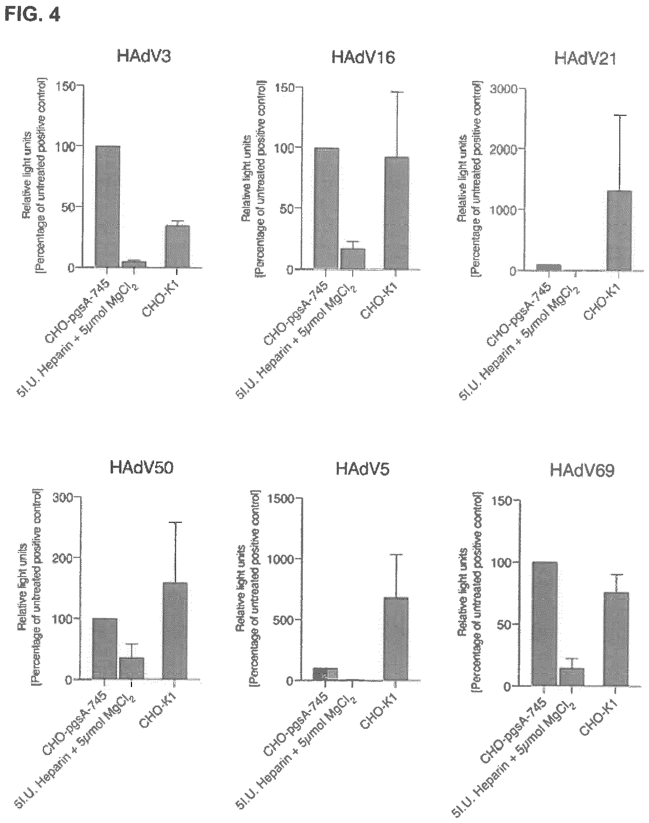

HeLa cells were infected with HAdV5 at 1000 vpc. Prior to infection (4 hrs) while plating the cells they were incubated with heparin and MgCl₂. GFP expression encoded by the adenoviral vector was measured. As displayed in Figure 4A at this viral dose GFP expression levels were saturated when analyzing the percentage of GFP positive cells regardless of the treatment regimen.

However, when calculating the median fluorescent intensity (MFI), significant differences between the different experimental groups were observed (Figure 4B). Cells treated with 5 I.U. heparin per well and MgCl₂ in an amount of 5 µmol per well showed a reduction to 30 % with respect to the MFI values. In sharp contrast, when compared to the groups which only received heparin or solely MgCl₂, only a slight fall in MFI to 93 % or a drop down to 41 % was measured, respectively.

### Example 4: CHO-pgsA-745 Cells Treated with Heparin and MgCl₂

Heparan sulfate-deficient (HSPG expression deficient) CHO-pgsA-745 cells were explored which are xylosyltransferase deficient (Esko, Stewart, & Taylor, 1985).. Cells were cultured using 5 % CO₂ and 37 °C in tissue culture dishes using DMEM medium (PAN-Biotech GmbH, Aidenbach, Germany) with 10% FCS (PAN-Biotech GmbH, Aidenbach, Germany) and 1% Penicillin/Streptomycin (PAN-Biotech GmbH, Aidenbach, Germany) until confluency was reached. Cells were harvested with trypsin-EDTA after washing them with DPBS. Cells were counted and seeded at a density of 3x10⁴ per well in 96-well tissue culture plates in triplicates for each test.

All luciferase measurements were performed 26 hrs post-infection. 50 µl medium of the cells were mixed with 50 µl solution from Nano-Glo® Luciferase Assay System. After five minutes incubation, the cell lysat was used for performing the luciferase measurement.

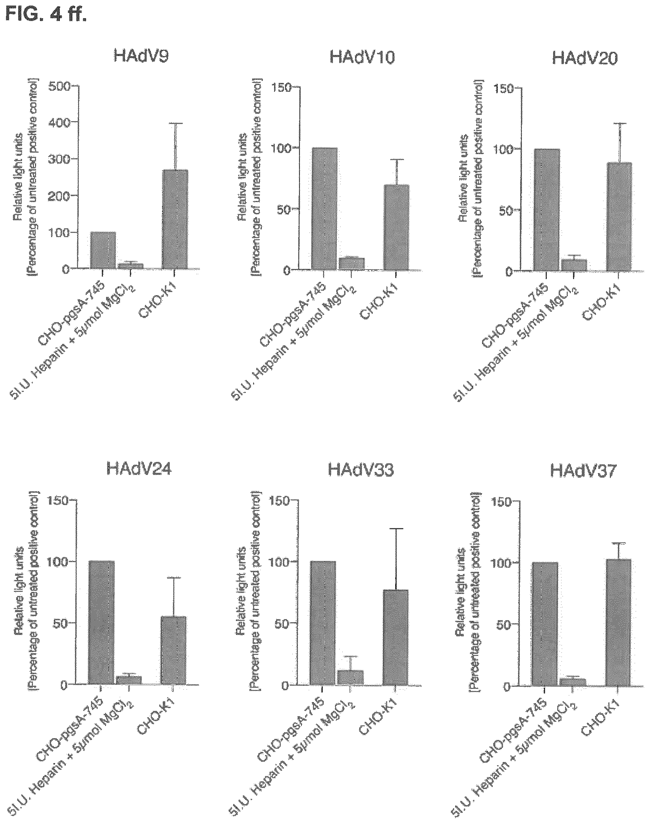

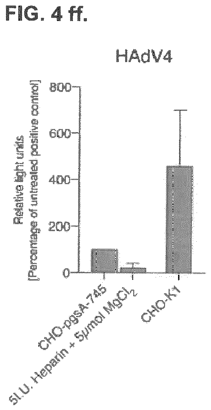

For each experiment untreated CHO-pgsA-745 cells (without heparin and MgCl₂) and CHO-K1 control cells were investigated. Different viral concentrations were used (200, 500, 1000, 2000, 5000 vpc). Note that for these experiments various HAdV types (HAdV16, -3, -14, -35, -5, -20, -9, -33, -10, -69, -24, -27, -24, -27 and -4) were used. To broadly explore the effect HSPG expression on HAdV infectivity, 12 HAdV types from the different HAdV species were analyzed on these cells. As control cells parental CHO cells (CHO-K1 cells) were explored. As displayed in Figure 4, higher luciferase levels were measured for HAdV24, - 69, -10, -33, -20, -16, and -3, hinting towards the hypotheses that for these viruses HSPG has an inhibitory effect on adenovirus activity. In contrast, lower virus derived luciferase expression levels were observed for HAdV4, -9, -5, and -50. Note that no effect on HAdV infectivity compared to the CHO-K1 cells was observed for HAdV37. Importantly there was a significant decrease in luciferase expression levels when cells were treated with heparin (5 I.U. per well) and MgCl₂ (5 µmol per well) before infection for all tested viruses (Figure 4). This effect was independent of whether the respective HAdV was heparan sulfate-dependent or not.

### Example 5: Binding Capacities of Anti-Adenoviral Antibodies to Heparin and MgCl₂ Adenovirus Coated Virions

To investigate the blocking effect of heparin and MgCl₂ coated HAdV virions and their influence on binding to anti-adenoviral antibodies an ELISA assay was established.

For the ELISA 96-well plates were used. The experiments were performed with human IVIG (intravenous immunoglobulin which consists of pooled and concentrated human IgGs) diluted 1:100 with PBS.

HAdV5 was incubated with heparin and MgCl₂ at the same concentrations as in the experiments before. 5 I.U. per well of heparin, 5 µmol per well MgCl₂ and 3x10⁶ virus particles, respectively, were added per well, i.e. per 100 µl. As a positive control, wells were coated with control virus only. As controls for unspecific binding affinities, wells were coated with MgCl₂ only or heparin in combination with coating buffer. After incubation of the different samples with coating buffer over night at 4°C, the plates were washed two times. In the next step, wells were blocked with blocking buffer for 45 min at room temperature. After washing five times IVIG (pre-diluted 1:100 with PBS) was added. IVIG was than successively diluted 1:2 in blocking buffer eleven times. The plate was incubated at 37 °C for one hour. After washing in blocking buffer, 1:2,000 diluted second antibody (Goat Anti-Human IgG H&L) was added to the wells and incubated at 37°C in a wet chamber for 70 minutes. Then the plate was washed five times again. After adding 100 µl substrate (OPD Peroxidase Substrate, Sigma, St. Louis, USA) solution to each well the plate was incubated in a dark chamber for 5 minutes. Finally, we added 100 µl H₂SO₄ per well to stop the reaction. The plate was measured with a Tecan ELISA Reader at a wavelength of 620 nm.

The evaluation of ELISA results was carried out with the statistic software GraphPad Prism (Version 7.04, GraphPad Software, San Diego, USA). After curve fit evaluation the dissociation constant (Kd) was determined according to the Michaelis-Menten model and drawn into the sigmoid curves.

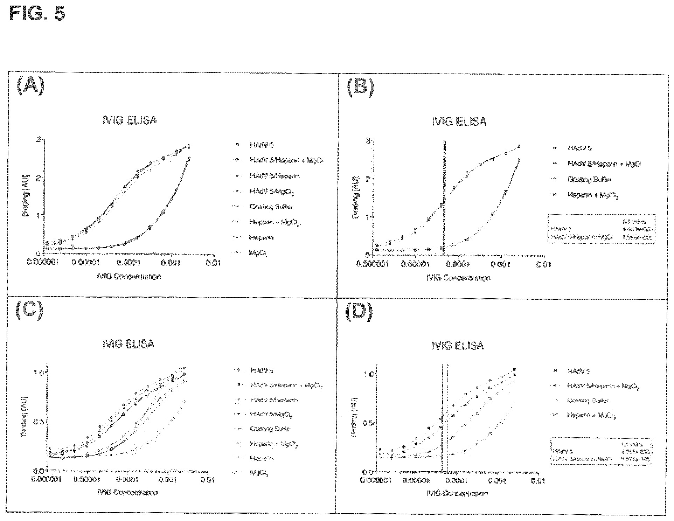

ELISA plates were coated with HAdV5 at two different densities (3x10⁷ and 3x10⁶ viruses per well) and the binding of pooled human IgGs (IVIG) was measured. As shown in Figure 5A and 5C a slight effect between the heparin and MgCl₂ treated viruses in IVIG binding capacity was observed. The heparin/MgCl₂ treated viruses show decreased optical densities correlating with blocked binding capacities of IVIG when a virus dose of 3x10⁷ was used. If the virus load per well is reduced to 3x10⁶ one can see a shift of the dissociation constant (Kd) to the right side if compared to the control group indicating that binding of heparin/MgCl₂ to the virion can influence antibody binding (Figure 5B and 5D).

### Example 6: Antiviral Effects of Different Heparin Concentrations in HSPG-deficient cells

The effect of heparin and heparin/ MgCl₂ was analyzed in further detail on HSPG-negative CHO-pgsA-745 cells by applying different heparin amounts and virus dosages (1,000 to 5,000 vpc). CHO-pgsA-745 cells (Xylotransferase-I deficient) and CHO-K1 cells were cultured under 5 % CO₂ and 37 °C in tissue culture dish until confluency was reached. The medium for treatment was DMEM medium (PAN-Biotech GmbH, Aidenbach, Germany) with 10 % FCS (PAN-Biotech GmbH, Aidenbach, Germany) and 1 % Penicillin/Streptomycin (PAN-Biotech GmbH, Aidenbach, Germany). Cells were washed with DPBS, harvested with trypsin-EDTA solution, counted and seeded at a density of 3x10⁴ per well in 96-well tissue culture plates in triplicates for each test. All luciferase measurements were performed 26 hrs after infection. 50 µl medium of the cells were mixed with 50 µl solution from Nano-Glo® Luciferase Assay System. After five minutes incubation, the cell lysate was used for performing the luciferase measurement. For each experiment positive controls (untreated cells) were included.

Different virus concentrations (200, 500, 1000, 2000, 5000 vpc) of HAdV5 and 50 were used.

Heparin was used at two different concentrations (5 I.U. and 50 I.U. per well). MgCl₂ was used in an amount of 5 µmol. Heparin and MgCl₂ were mixed with the culture medium in advance and added to the cells. After cell attachment viruses were added to the cells.

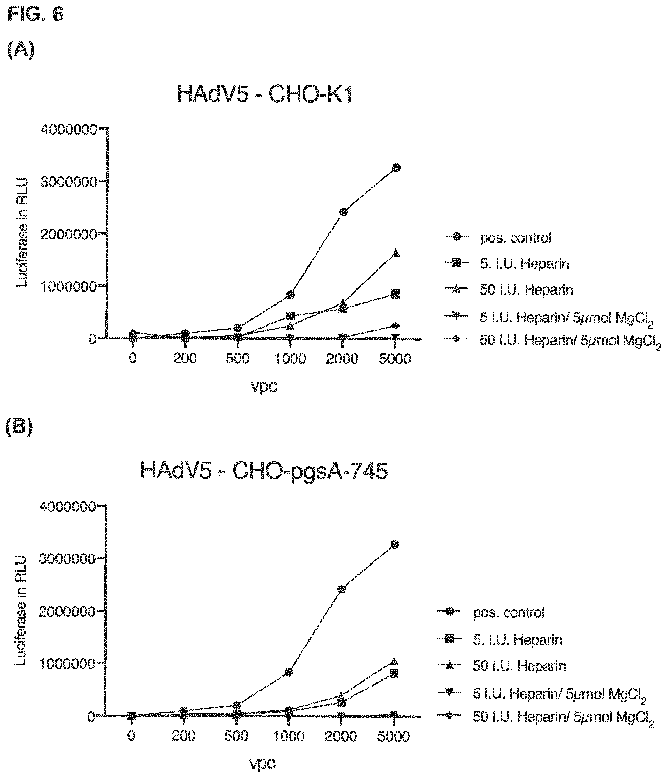

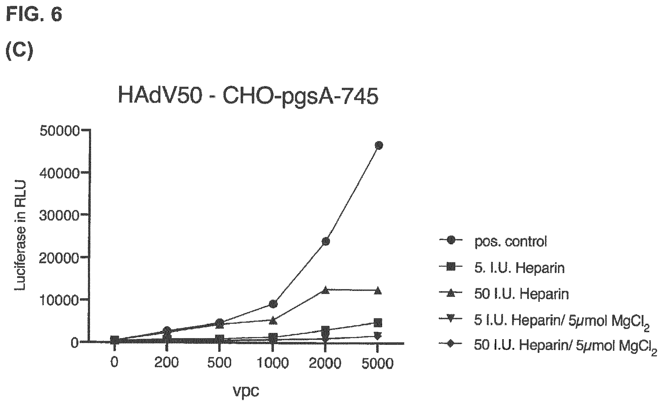

The different amounts of heparin and MgCl₂ were added to the culture medium before infection. Figure 6 shows that solely heparin resulted in a dose-dependent decrease in luciferase expression levels. The high amount of heparin with 50 I.U. per well showed a less pronounced effect compared to the lower amount of heparin, independent of which cell line or virus were used (HAdV5 and HAdV50) (Figure 6).

Importantly, in the presence of MgCl₂ (5 µmol) a general decrease of HAdV-derived luciferase expression levels independent of the cell line and the virus used can be measured. Note that the differences of viral transduction efficiencies of high (50 I.U.) or low (5 I.U.) applied heparin saturated with MgCl₂ (5 µmol) is visible but low (Figure 6).

### Example 7: A Direct Comparison of HAdV Infection in CHO-pgsE-606 and CHO-pgsA-745 Cells

As a further step, sulfation-deficient CHO cells (CHO-pgsE-606) were explored that are Heparan sulfate N-sulfotransferase deficient (Esko et al., 1985) and directly compared to the biological properties of HAdV in CHO-pgsA-745 cells.

CHO-pgsA-745 (Xylotransferase-I deficient) and CHO-pgsE-606 cells (partially deficient in heparan sulfate N sulfotransferase) were cultured by applying 5 % CO₂ and 37 °C in tissue culture dishes in DMEM medium (PAN-Biotech GmbH, Aidenbach, Germany) supplemented with 10 % FCS (PAN-Biotech GmbH, Aidenbach, Germany) and 1 % Penicillin/Streptomycin (PAN-Biotech GmbH, Aidenbach, Germany). Cells were harvested with trypsin-EDTA after washing them with DPBS. Cells were counted and seeded at a density of 3x10⁴ per well in 96-well tissue culture plates.

Subsequently, luciferase measurements were performed 26 hrs after infection by mixing 50 µl medium of the cells with 50 µl solution from the Nano-Glo® Luciferase Assay system. For each experiment positive untreated controls were performed. Differing virus concentrations (200, 500, 1,000, 2,000, 5,000 vpc) of HAdV 16, 3, 14, 35, 5, 20, 9, 33, 10, 69, 24, 27 and 4 were used.

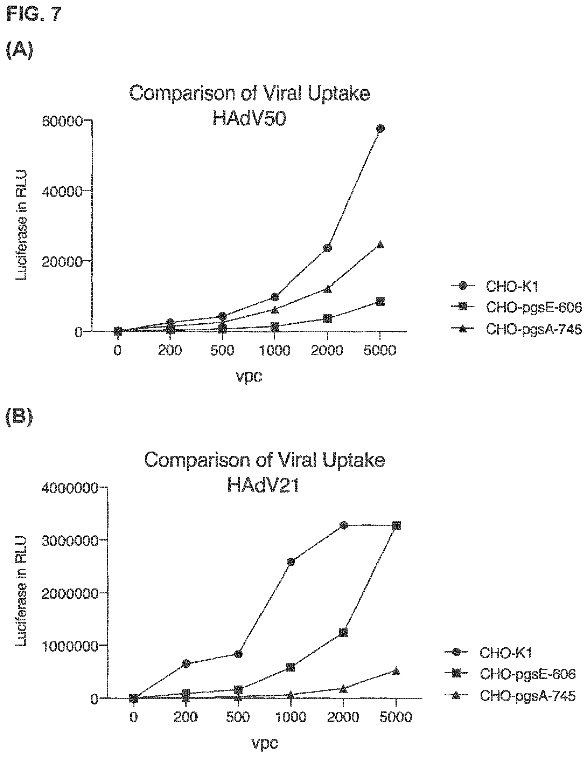

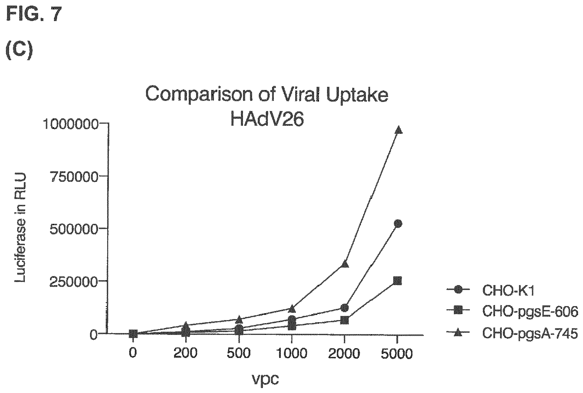

In concordance with experiments shown in Figure 7 decrementally increased amounts of virus dosages were applied and luciferase expression levels were measured 26 hrs post-infection. Here it was found that HAdV50 and HAdV21 showed decreased luciferase expression levels for both cell lines, whereas HAdV26 resulted in a decreased luciferase production after infection in case of CHO-pgsE-606 but not in the case of CHO-pgsA-745 cells (Figure 7A-C). Furthermore, for hAdV50 luciferase expression levels were higher in CHO-pgsE-606 cells and in case of HAdV21 the opposite effect was observed (Figure 7).

### Example 8: Competition Assay with Factor X and Heparin/MgCl₂ Covered Virions

To analyze whether there is a competition between FX (Coagulation Factor X) binding and the combination of heparin/MgCl₂ regarding binding to the virion, competition assays were performed. For these experiments SKOV3 cells were utilized which are CAR-deficient.

SKOV3 cells were cultivated in RPMI with 5% FCS, 1% penicillin/streptomycin/glutamine. For the experiments they were seeded in 96-well plates. Each well contained 2x10⁴ cells with 200 µl medium. The following day the medium was removed and cells were washed once with 100 µl PBS and supplied with 100 µl serum-free medium. The final concentration of FX was 8 µg/ml. The virus load was set to 1,000 vpc. The MgCl₂ amount was set to 5 µmol per well and the heparin amount to 5 I.U. per well.

In this experiment five groups were set up and performed in triplicate, respectively. HAdV5 only was used as the positive control. In the next group the virus was mixed with FX and added to the cells. In the third group FX was added after the virus was mixed with heparin and magnesium chloride and incubated for 15 minutes. In the last row, after the virus was incubated with FX for 15 minutes by room temperature, the mixture of heparin and MgCl₂ was added. After adding the viruses to the cells, the cells were incubated for 5 hours at 37 degrees. Then the medium was removed from the cells and washed once with PBS. Subsequently 200 µl serum-free medium was added to the cells. Then the cells were incubated at 37 °C for 72 hours. On the fifth day luciferase measurements were performed. For the measurement the protocol for Glo® Luciferase Assay system was utilized.

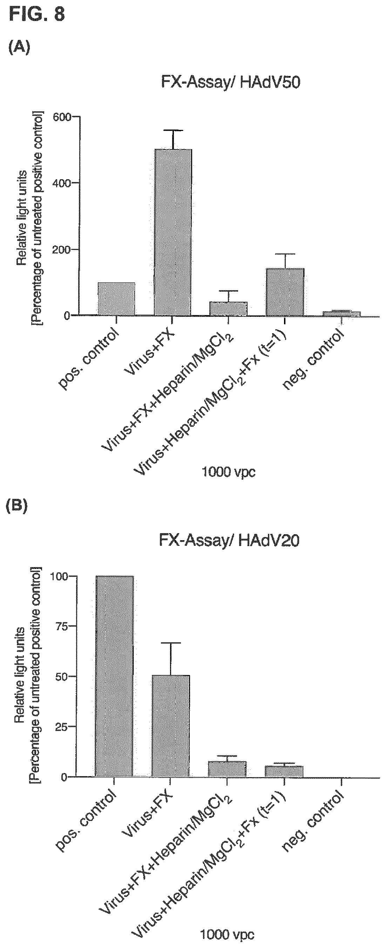

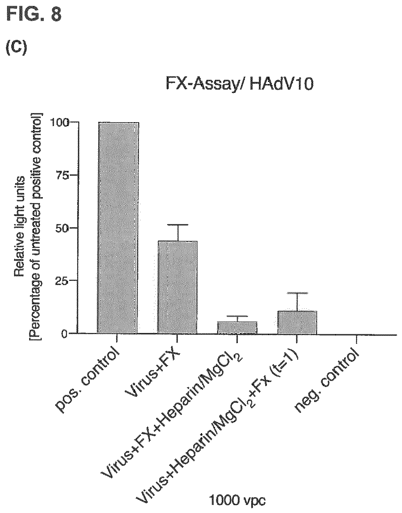

In case of HAdV50 which are known to possess high binding affinities to FX (Waddington et al., 2008), higher luciferase expression levels after incubation with FX were observed (Figure 8). Furthermore, HAdV20 with no binding affinities to FX (Waddington et al., 2008) and HAdV10 with unknown FX binding affinities were explored. In case of HAdV20 and HAdV10 lower luciferase lower values were measured after incubation with FX (Figure 8B and 8C). In the case of HAdV50 higher luciferase levels were quantified when the virus was mixed with heparin / MgCl₂ pre-infection (Figure 8A). We observed the opposite effect when performing experiments with HAdV20 and HAdV10 (Figure 8B and 8C).

### Example 9: Different Conditions of Treating the Cells with HAdV, Heparin and MgCl₂

To further analyze the potential mechanism of the observed phenomenon we explored different infection and incubation conditions.

For these experiments CHO-K1 cells were used cultured in DMEM medium (PAN-Biotech GmbH, Aidenbach, Germany) with 10 % FCS (PAN-Biotech GmbH, Aidenbach, Germany) and 1 % Penicillin/Streptomycin (PAN-Biotech GmbH, Aidenbach, Germany). Cells were harvested with trypsin after washing them with DPBS. Subsequently, cells were counted and seeded at a density of 3x10⁴ per well in 96-well tissue culture plates in triplicate for each test. All luciferase measurements were performed 26 hours after infection with HAdV5 and HAdV10 (1,000 vpc). Heparin and MgCl₂ were applied in stable amounts of 5 I.U. heparin per well and 5 µmol MgCl₂ per well.

26 hrs post-infection a luciferase assay was performed. 50 µl medium of the cells were mixed with 50 µl solution from Nano-Glo® Luciferase Assay System.

It was analyzed whether the time point of adding heparin and/or MgCl₂, i.e. before or after virus infection, influences virus infectivity and the inhibitory effect of these reagents (heparin 5 I.U. per well and MgCl₂ 5 µmol per well).

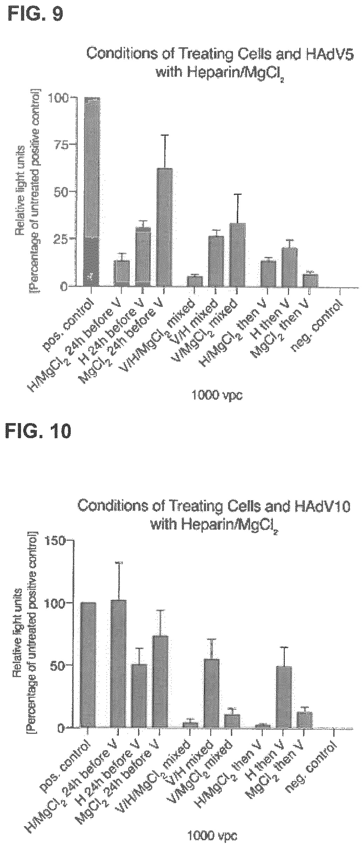

In case of HAdV5 a significant decrease of luciferase expression levels down to 14 % was measured, if one mixes heparin and MgCl₂ and applying them to the cells before infection with the virus (Figure 9). By administering heparin alone, one achieves a reduction of luciferase production to about 31 % (Figure 9). If MgCl₂ was mixed with the cells before adding the remaining components (virus and heparin), a reduction of virus infectivity to 61 % was achieved. If one adds the virus 15 minutes after incubation with heparin and MgCl₂, a reduction of luciferase expression levels down to 13 % was measured. When mixing all three components simultaneously and then infecting the cells, a drop down to about 5 % was measured. After incubating virus and heparin and then infecting the cells, one can obtain a drop down to 26 %. When only applying virus and MgCl₂ a decrease to about 32 % was seen. When heparin and MgCl₂ were incubated before and the virus added thereafter, the bar fell to 14 %. When heparin was added to the medium and then the virus was added, the bar fell to 20 %. When only using MgCl₂ and virus, luciferase expression levels decrease down to 7 % compared to untreated cells solely infected with virus.

In the case of HAdV10 only a small effect of 3 % was observed when heparin (5 I.U. per well) and MgCl₂ (5 µmol per well) were mixed and then applied to the cells before infecting with the virus (Figure 10). For HAdV10 the administration of heparin alone reduced luciferase production down to 48 % (Figure 10). If MgCl₂ was mixed with the cells before adding the remaining components, a reduction down to 74 % was measured. When the virus was added 15 minutes after incubation of cells with heparin and MgCl₂, a reduction down to 59 % was measured. When all three components were mixed simultaneously before infecting the cells, the HAdV10 infectivity dropped down to 4 %. Incubating virus and heparin before infecting the cells led to a drop down to 52 %. When only applying virus and MgCl₂ a drop to 10 % is seen, and when incubating heparin and MgCl₂ before and then adding the virus, the bar fell to 2 %. When mixing the medium, heparin and the virus the bar decreases to 47 %. With only MgCl₂ and virus luciferase expression levels decrease to 12 % (Figure 10). All results of these experiments are summarized in Figure 10, which demonstrates that also for HAdV10 the setup and timing of mixing and matching the components are important.

### Example 10: Antiviral Effect of Heparin and Magnesium Chloride on HSV-1 infected Vero Cells

The infectivity of the HSV-1 virus suspension was investigated using a concentration gradient (0 to 60 µl HSV-1 suspension). A dose of 40 µl on 1.5x10⁵ Vero cells resulted in -90% GFP-positive cells as measured by flow cytometry. A dose of 40 µl was applied for the following experiments. Aciclovir (Aciclovirratiopharm, ratiopharm GmbH, Ulm, Germany) diluted in Ampuwa® water for injection was used as a control to compare the effect of Heparin/MgCl₂ with the standard medication. A concentration gradient between 0 and 3 µg/ml acyclovir was evaluated. 2.5 µg/ml acyclovir was used for the following experiments.

For antiviral treatment, 40 µl virus suspension was mixed with 5 I.U. Heparin and 5 µmol MgCl₂ directly and then added to Vero cells. After infection, the cells were cultured 24 h under a humidified atmosphere of 5 % CO₂ and 37 °C.

The transduction efficiencies of tagged HSV-1 in Vero cells were measured by determining the reporter gene (GFP) expression levels. 24 h after infection flow cytometry analyses (Navios Flow Cytometer, Beckmann Coulter, Krefeld, Germany) were performed. The percentage of GFP-positive cells and die mean fluorescence intensity (MFI) in arbitrary units (AU) were evaluated. All experiments were performed in triplicates and repeated three times.

In another experiment, different treatment periods were also used: heparin/MgCl₂ treatment during seeding of the cells; heparin/MgCl₂ simultaneously with infection of the cells; heparin/MgCl₂ one hour after infection with HSV-1.

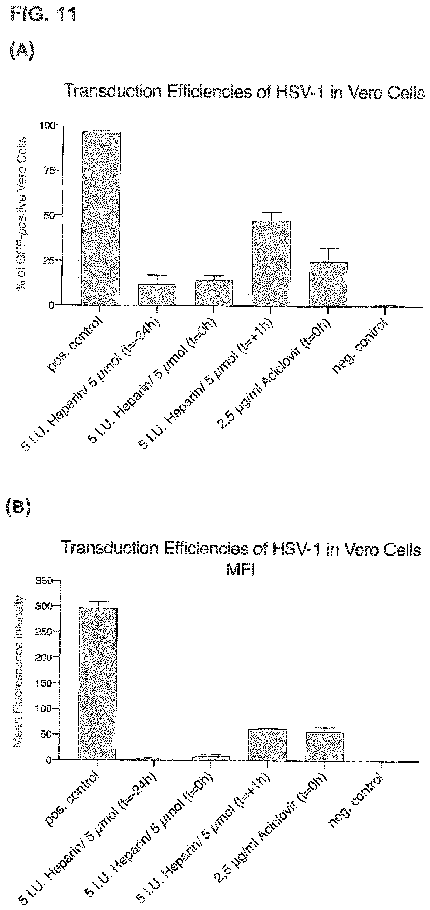

To investigate the effect of Heparin and MgCl₂ on HSV-1, 40 µl of virus suspension diluted in a total of 1 ml of medium (DMEM, PAN Biotech, Aidenbach, Germany) were treated at different time points during the experiments (t=0, when seeding the cells; t= +1, one hour after infection). We found that treatment with Heparin/MgCl₂ significantly decreased GFP expression levels derived from incoming HSV-1 genomes in Vero cells (Figures 11A and B). A lower GFP expression was found too, compared to acyclovir control. The differences could be shown regarding the quantification of GFP-positive cells as well as the evaluation of the mean fluorescence intensity.

### Example 11: Antiviral Effect of Heparin and Magnesium chloride on H9N2 Influenza Virus

For the immunostaining with MDCKII cells infected with H9N2, 3x10⁵ cells were seeded with DMEM and 5 % FBS, 1% Na-Pyruvat (Sodium pyruvate, PAN Biotech, Aidenbach, Germany) and NEAA (MEM Non-essential Amino Acid Solution, PAN Biotech, Aidenbach, Germany) in a 96 well plate. Before infection, medium was removed, cells were washed once with PBS before substitution with DMEM supplemented with 5 % BSA with 1 µg/ml TPCK-trypsin. Cells were infected with H9N2at MOI 0,1 and treated with 5 I.U heparin per well and 5 µmol MgCl₂ per well. After one hour incubation in the incubator (5 % CO₂ and 37°C), medium was removed again, cells were washed with DPBS and cultured with the medium as described before for 18 hours.

Subsequently the supernatant was pooled from each triplicate and 50 µl were subjected to RNA isolation for RT-PCR examination.

Cells in the plates were washed once with DPBS. Cells were fixed with 80% acetone for 10 minutes at -4 °C. Next, cells were washed with DPBS and blocked with 5% BSA for 30 minutes by 37°C.

The influenza rabbit anti-nucleoprotein (NP) polyclonal primary antibody (PA5-32242, Thermo scientific, Germany) was diluted 1:750 in 5% BSA and added to the cells. After incubation for 1 hour by 37°C cells were washed twice with BSA (5%).

The secondary antibody (Alexa Fluor® 488 Goat Anti-Rabbit IgG, Life technologies, Germany) was diluted 1:1,000 in 5% BSA and added to each well. For cell core staining 4, 6-diamidino-2-phenylindole (DAPI) 1:1,000, was used. Each plate was incubated for 1 hour at 37 °C. After incubation, wells were washed with 5 % BSA three times and filled with 100 µl DPBS.

For the immuno-fluorescence imaging (Olympus IX70, Tokyo, Japan), the center of each well was selected and images of Alexa Fluor 488 (green) and DARPI (blue) were taken. An overlay of the individual images was then performed.

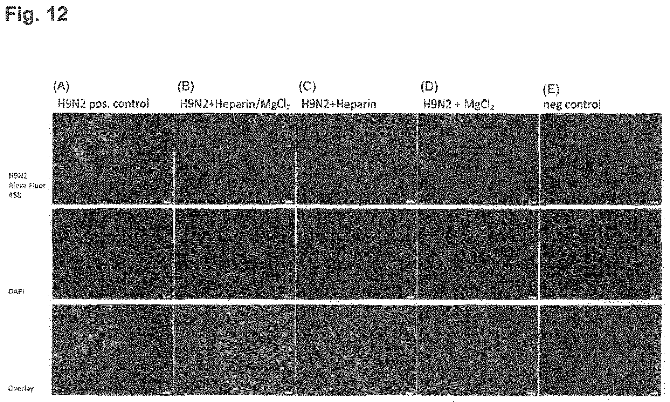

Figure 12 shows the staining results from the wells in which 2x10⁴ MDCKII cells were seeded per well. Figure 12A shows cells infected with H9N12 as an untreated positive control. Figure 12B shows the infected cells treated with Heparin (5 I.U. per well) and MgCl₂ (5 µmol per well). Figure 12C depicts cells treated with Heparin 5 I.U. per well before infection, Figure 12D shows cells treated with MgCl₂ (5 µmol per well) before infection. Figure 12E shows the untreated negative control.

A significant decrease of infected cells was observed in Figure 12B referring to infected cells treated with Heparin (5 I.U. per well) and MgCl₂ (5 µmol per well). A slight decrease was detected for groups depicted in Figure 12C and 12D. The overlay data from each panel shows the cell-nucleus-related infection rate of the cells.

## Claims

1. Pharmaceutical or veterinary composition, comprising heparin and one or more pharmaceutically acceptable magnesium salts.

2. Pharmaceutical or veterinary composition according to claim 1 which is a solution in water or in a suitable organic solvent or a mixture thereof.

3. Pharmaceutical or veterinary composition according to claim 1 or 2, wherein the magnesium salt is one or more of chloride, bromide, fluoride, iodide, sulfate, hydrogen sulfate, phosphate, hydrogen phosphate, dihydrogen phosphate, nitrate, fumarate, oxalate, maleate, malate, citrate, oxalate, succinate, lactate, glycolate, aspartate, mesylate, tosylate, preferably magnesium chloride.

4. Pharmaceutical or veterinary composition according to one or more of claims 1 to 3, wherein heparin is one or more of unfractionated heparins (UFH) having a Mw 4,000 to 40,000 Da or low-molecular weight heparins (LMWH) having a Mw of 4,000 to 6,000 Da.

5. Pharmaceutical or veterinary composition according to one or more of claims 1 to 4, which is in form of a solution for injection, gel, cream, ointment, spray, aerosol, tincture, eyedrops, nose drops, nose spray, solution or lotion.

6. Pharmaceutical or veterinary composition according to one or more of claims 1 to 5, wherein the ratio of heparin to magnesium salt is in the range of 25 I.U heparin : 1 µmol magnesium salt to 125 I.U. heparin : 10 µmol magnesium salt, preferably 5 I.U. heparin : 5 µmol magnesium salt.

7. Pharmaceutical or veterinary composition according to any of claims 1 to 6, wherein the concentrations of heparin and magnesium salt are 0.22 I.U./ml to 1.13 I.U./ml heparin and 0.9 µmol/ml to 9 µmol/ml magnesium salt, respectively, preferably 0.45 I.U./ml heparin and 4.5 µmol/ml magnesium salt, respectively, based on the total composition.

8. Pharmaceutical or veterinary composition according to one or more of claims 1 to 5, or combination of heparin and one or more pharmaceutically acceptable magnesium salts, for use in a prophylactic or therapeutic method for treating virus infections.

9. Pharmaceutical or veterinary composition for the use according to claim 8, wherein the prophylactic or therapeutic treatment is a topical or an intravenous treatment.

10. Combination for the use defined in claim 8, where heparin and the magnesium salt are used concomitantly or consecutively in any order.

11. Pharmaceutical or veterinary composition or combination for the use as defined in any of claims 8 to 10, wherein the virus infection is caused by any double-stranded RNA- or DNA-viruses which are enveloped or non-enveloped.

12. Pharmaceutical or veterinary composition or combination for the use as defined in any of claims 1 to 11, wherein the virus infection is caused by adenovirus, herpes simplex virus or influenza virus.

13. Pharmaceutical or veterinary composition or combination for the use as defined in any of claims 8 to 10, wherein the virus infection is caused by adenovirus.

14. Pharmaceutical or veterinary composition or combination according to any of claims 8 to 13, wherein the virus infection is a respiratory tract disorder selected from cold, bronchitis, pneumonia, acute respiratory distress syndrome, gastroenteritis, keratoconjunctivitis epidemica, cystitis, rhinitis, pharyngitis or diarrhea.
